(19) Europäisches Patentamt
European Patent Office
Office européen des brevets

(11) Numéro de publication : **0 245 156 B1**

(12) **FASCICULE DE BREVET EUROPÉEN**

(45) Date de publication du fascicule du brevet : **03.01.90**

(51) Int. Cl.$^5$ : **C 07 C 69/736**, C 07 C 67/31

(21) Numéro de dépôt : **87400967.3**

(22) Date de dépôt : **27.04.87**

(54) **Nouveau procédé de préparation de fibrates.**

(30) Priorité : **30.04.86 FR 8606258**

(43) Date de publication de la demande : **11.11.87 Bulletin 87/46**

(45) Mention de la délivrance du brevet : **03.01.90 Bulletin 90/01**

(84) Etats contractants désignés : **AT BE CH DE ES FR GB GR IT LI LU NL SE**

(56) Documents cités :
EP-A- 0 033 980
EP-A- 0 108 592
AT-B- 367 390

(73) Titulaire : **FOURNIER INNOVATION ET SYNERGIE
38, avenue Hoche
F-75008 Paris (FR)**

(72) Inventeur : **Bourgogne, Jean-Pierre
17 rue du Bief du Moulin
F-21600 Longvic (FR)**
Inventeur : **Sornay, Roland
Le Pré de la Croix Brognon
F-21490 Ruffey les Echirey (FR)**

(74) Mandataire : **Clisci, Serge et al
S.A. FEDIT-LORIOT CONSEILS EN PROPRIETE INDUSTRIELLE 38, avenue Hoche
F-75008 Paris (FR)**

**EP 0 245 156 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention est relative à un nouveau procédé de préparation de fibrates.

On désigne par fibrates une famille de composés présentant des propriétés hypocholestérolémiante et hypolipidémiante et répondant à la formule générale :

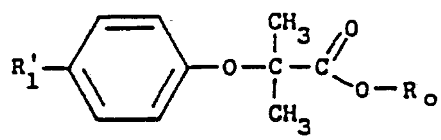

(I)

où $R'_1$ représente notamment un atome d'halogène, un groupe 2,2-dichloro-cyclopropyle, un groupe (4-chlorophényl) hydroxyméthyle, un groupe 4-chlorobenzoyle, ou un groupe 2-(4-chlorobenzamido)-éthyle et $R_0$ représente un atome d'hydrogène ou un groupe alkyle en $C_1$-$C_4$ ramifié ou non.

Dans cette famille, on connaît notamment (i) le clofibrate, qui a pour nomenclature : ester éthylique de l'acide 4-chlorophénoxy-2-méthylpropanoïque ou 2-(4-chlorophénoxy)-2-méthylpropanoate d'éthyle, et (ii) le fénofibrate qui a pour nomenclature : 1-méthyléthyl ester de l'acide 2-(4-(4-chlorobenzoyl) phénoxy)-2-méthylpropanoïque ou 2-(4-(4-chlorobenzoyl)-phénoxy)-2-méthylpropanoate d'isopropyle.

On sait que l'on a préconisé dans le passé différentes méthodes de synthèse des fibrates. Le document de brevet GB-A-860 303, relatif à l'obtention du clofibrate, propose de faire réagir un phénol de formule $4\text{-}ClC_6H_4OH$ sur un mélange acétone/chloroforme en présence de soude puis d'estérifier l'acide ainsi obtenu au moyen d'alcool éthylique.

Le document de brevet GB-A-1 415 295, relatif à l'obtention du fénofibrate, propose un procédé, analogue à celui de GB-A-860 303 précité, et qui comprend les étapes suivantes :

(a) réaction d'un mélange acétone/chloroforme avec la (4-chlorophényl)-(4-hydroxyphényl)-méthanone,

(b) transformation de l'acide obtenu suivant ladite réaction en chlorure d'acide, puis

(c) estérification par réaction de l'alcool isopropylique sur ledit chlorure d'acide.

Par ailleurs, GB-A-1 539 897 indique qu'il est possible d'accéder aux composés de formule

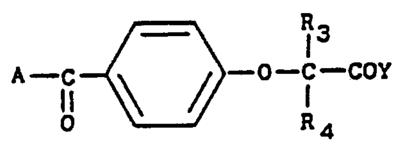

(II)

où, en particulier, A est un radical phényle substitué par un atome d'halogène, $R_3$ et $R_4$, identiques ou différents, représentent chacun l'atome d'hydrogène ou un groupe alkyle, et Y représente un groupe hydroxy ou un groupe alkoxy, d'une part, selon la méthode dite « acétone/chloroforme » mettant en œuvre ledit mélange acétone-chloroforme, d'autre part, par condensation dans un solvant approprié d'un phénol substitué de formule

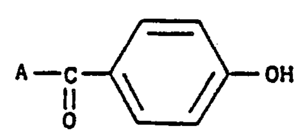

(III)

avec un dérivé bromé de formule

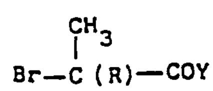

(IV)

Selon la nature du groupe R que l'on souhaite obtenir dans le produit final, notamment à partir du dérivé d'acide 2-bromopropanoïque de formule IV ci-dessus comportant ledit groupe R, il est plus particulièrement recommandé dans GB-A-1 539 897 :

(i) de ne pas utiliser la réaction III + IV, lorsque R est $CH_3$, mais de mettre en œuvre la méthode dite « acétone/chloroforme » pour l'obtention d'un dérivé d'acide 2-phénoxy-2-méthylpropanoïque appartenant à l'ensemble des fibrates, et

(ii) de mettre en œuvre la réaction III + IV, lorsque R est H, pour l'obtention d'un dérivé d'acide 2-phénoxypropanoïque, ladite réaction du phénol III avec le dérivé bromé IV étant réalisée dans un solvant organique, tel que l'éthanol ou la méthylisobutylcétone, en présence de $K_2CO_3$.

Ainsi, selon la description de GB-A-1 539 897, on obtient le 2-(4-(4-chlorobenzoyl)-phénoxy)-propionate d'éthyle avec un rendement de 76 % lorsque l'on fait réagir dans des proportions environ molaires, le 2-bromopropanoate d'éthyle (i.e. le composé de formule IV où R = H et $Y = OCH_2CH_3$) avec la (4-chlorophényl)-(4-hydroxyphényl)-méthanone [i.e. le composé de formule III où A est $4\text{-}ClC_6H_4$ et qui

répond également à la nomenclature de 4-(4-chlorobenzoyl)-phénol] dans la méthylisobutylcétone en présence de $K_2CO_3$.

On connaît par ailleurs de AT-A-367 390 un procédé de préparation de dérivés d'acide 2-(3-phénoxyphénoxy)-propanoïque, où les groupes phényle sont substitués notamment par des atomes d'halogène, selon un mécanisme réactionnel sans solvant. Selon AT-A-367 390, on prépare notamment le 2-[(6-chloro-3-(2,4-dichlorophénoxy)) phénoxy]-propanoate de méthyle par réaction, en l'absence de solvant, du 6-chloro-3-(2,4-dichlorophénoxy)-phénol avec le 2-bromopropanoate de méthyle en présence de $K_2CO_3$. La comparaison des rendements de cette réaction mise en œuvre avec un solvant (méthanol) [rendement 76 %] selon l'enseignement de GB-A-1 539 897, ou sans solvant selon AT-A-367 390 [rendement 72 %] montre qu'il n'y a pas de différences significatives entre la technique avec solvant et la technique sans solvant.

Selon l'invention, on préconise une nouvelle technique pour résoudre le problème de la synthèse des fibrates. Cette technique, qui conduit à des rendements nettement supérieurs à ceux de l'art antérieur le plus proche, va de façon surprenante à l'encontre de l'enseignement de GB-A-1 539 897 en ce sens qu'elle met en œuvre la réaction d'un dérivé bromé de formule IV où R est $CH_3$ avec un phénol de formule III, en l'absence de solvant, d'une part, et à l'encontre de l'enseignement de AT-A-367 390 en ce sens qu'elle améliore les rendements de façon significative, d'autre part.

Le procédé de préparation selon l'invention pour la préparation d'un fibrate de formule

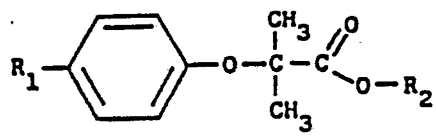

(I)

dans laquelle $R_1$ représente notamment un atome d'halogène (en particulier F, Cl, Br, l'halogène préféré étant Cl), un groupe acétyle, un groupe (4-chlorophényl)-hydroxyméthyle de formule $4\text{-}ClC_6H_4CH(OH)$, un groupe 4-chlorobenzoyle ou un groupe 2-(4-chlorobenzamido)-éthyle, et $R_2$ représente un groupe alkyle en $C_1$-$C_4$ à chaîne hydrocarbonée linéaire ou ramifiée, est caractérisé en ce que l'on fait réagir, en l'absence de solvant, un excès par rapport aux conditions stœchiométriques de 2-bromo-2-méthylpropanoate d'alkyle de formule :

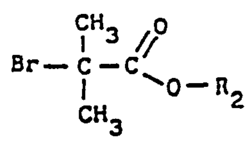

(V)

où $R_2$ est défini comme indiqué ci-dessus, avec un phénol substitué de formule

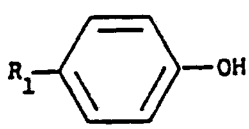

(VI)

où $R_1$ est défini comme indiqué ci-dessus, en présence d'un excès de $K_2CO_3$ par rapport aux conditions stœchiométriques, à une température supérieure ou égale à 120 °C, pendant au moins 2 heures.

Selon un mode de mise en œuvre de ce procédé, le fibrate ainsi obtenu est isolé directement du milieu réactionnel par précipitation, extraction ou distillation.

Selon un autre mode de réalisation, le milieu réactionnel contenant le fibrate provenant de la réaction V + VI est traité par un acide fort (notamment HCl, $H_2SO_4$) pour neutraliser l'excès de $K_2CO_3$, le fibrate ainsi obtenu étant ensuite isolé du milieu réactionnel résultant par précipitation, extraction ou distillation.

Pour isoler le fibrate obtenu selon le procédé de l'invention, on procède à (i) une opération de précipitation lorsque ledit fibrate est un solide (c'est le cas du fénofibrate et de ses analogues de formule I ci-dessus), ou (ii) une opération d'extraction au moyen d'un solvant approprié ou une opération de distillation lorsque ledit fibrate est liquide ou huileux (c'est le cas du clofibrate).

Les conditions stœchiométriques comprennent la réaction de 1 mole de VI ave 1 mole de V en présence de 0,5 mole de $K_2CO_3$. Comme indiqué ci-dessus, on réalise la réaction VI + V de telle façon que le dérivé bromé V et $K_2CO_3$ soient en excès par rapport auxdites conditions stœchiométriques. De façon avantageuse, l'on fera réagir à une température de 120 à 160 °C, pendant 3 à 6 heures, 1 mole de phénol substitué de formule VI avec environ 1,7 à environ 2,3 moles de dérivé de formule V, en présence d'environ 0,8 à environ 1,8 moles de $K_2CO_3$.

Quand elle est effectuée, la neutralisation de l'excès de $K_2CO_3$ par un acide fort est réalisée à une température inférieure ou égale à 120 °C et de préférence à une température voisine de 100 °C. L'acide fort est avantageusement un acide minéral tel que HCl et mieux $H_2SO_4$.

En bref, le procédé selon l'invention pour préparer un ester de formule I comprend les deux ou trois étapes qui suivent :

1) l'on fait réagir, en l'absence de solvant, pendant au moins 2 heures (de préférence pendant 3 à 6 heures), environ une mole de VI avec environ 1,7 à environ 2,3 moles de V (de préférence environ 2 moles de V), en présence de environ 0,8 à environ 1,8 moles de $K_2CO_3$ (de préférence environ 1 mole de $K_2CO_3$), à une température de 120 °C à 160 °C (de préférence à une température de 140 °C à 145 °C),

2) le cas échéant, on neutralise l'excès de $K_2CO_3$ par un acide fort à une température inférieure à 120 °C, et

3) on isole le fibrate du milieu réactionnel par précipitation à une température inférieure à 60 °C, par extraction ou par distillation.

Le meilleur mode de mise en œuvre que l'on préconise pour préparer le fénofibrate suivant le procédé selon l'invention consiste à :

(a) faire réagir, en l'absence de solvant, pendant 5 heures, environ 1 mole de VI où $R_1$ est le groupe 4-chlorobenzoyle avec environ 2 moles de V où $R_2$ est le groupe isopropyle, en présence de environ 1 mole de $K_2CO_3$, à une température de environ 140 °C à environ 145 °C,

(b) après addition d'isopropanol aqueux au milieu réactionnel ainsi obtenu, neutraliser l'excès de $K_2CO_3$ par l'acide sulfurique à une température voisine de 100 °C,

(c) refroidir le milieu réactionnel à une température comprise entre 15 et 25 °C et recueillir le précipité de fénofibrate par filtration,

(d) laver le précipité de fénofibrate ainsi recueilli successivement à la soude puis à l'eau, et

(e) recristalliser le fénofibrate de l'isopropanol aqueux.

Le procédé selon l'invention est également applicable à la préparation des fibrates, qui, tel le bézafibrate, présentent un groupe acide carboxylique $R_0 = H$ au lieu d'un groupe carboxylate. Toutefois, eu égard au rendement de la réaction phénol VI + dérivé bromé V dans lequel $R_2$ serait H, on préfère plutôt opérer en deux stades, à savoir : préparation de l'ester correspondant selon le procédé de l'invention à partir d'un dérivé bromé V où $R_2$ est un groupe alkyle puis saponification dudit ester pour obtenir l'acide souhaité.

Dans le tableau I qui suit, on a consigné les résultats des essais comparatifs qui ont été entrepris pour montrer l'intérêt du procédé de l'invention (Ex 1) sans utilisation d'un solvant pour la réaction V + VI, par rapport à l'utilisation de la même réaction avec un solvant (CP1-CP4) selon l'enseignement de GB-A-1 539 897, pour la synthèse du fénofibrate. Dans le tableau I, on a également mentionné par commodité les rendements de la préparation du fénofibrate selon la méthode dite « acétone/chloroforme » (CP6) et du 2-(4-(4-chlorobenzoyl)-phénoxy)-propanoate d'éthyle (CP5) selon la réaction III + IV où R est H en présence d'un solvant. Les solvants utilisés dans les exemples comparatifs CP1 et CP2 sont ceux mentionnés spécifiquement dans GB-A-1 539 897, les solvants utilisés dans les exemples comparatifs CP3 et CP4 sont inclus dans l'enseignement de GB-A-1 539 897 sans être toutefois spécifiquement exemplifiés dans ledit document.

L'invention sera mieux comprise à la lecture de la description qui va suivre d'un exemple de préparation selon le procédé préconisé ici et d'exemples comparatifs selon l'art antérieur le plus proche (GB-A-1 539 897), pour l'obtention du fénofibrate, ainsi que des exemples de préparation d'autres fibrates.

Préparation I : (Exemple 1)

Obtention du 1-méthyléthyl ester de l'acide 2-(4-(4-chlorobenzoyl)-phénoxy)-2-méthyl-propanoïque (fénofibrate)

Dans un réacteur de 4 litres équipé d'une agitation et d'un réfrigérant, on introduit 465 g (2 moles) de (4-chlorophényl)-(4-hydroxyphényl)-méthanone et 815 g (3,9 moles) de 1-méthyléthyl ester de l'acide 2-bromo-2-méthylpropanoïque (autre nomenclature : 2-bromo-2-méthylpropanoate d'isopropyle). On porte le milieu à 120 °C, puis on ajoute 265 g (1,92 moles) de carbonate de potassium à l'aide d'une ampoule à solide. On chauffe ensuite pendant 5 heures à 140-145 °C, puis on refroidit le milieu réactionnel jusque vers 100 °C. Le milieu réactionnel est ensuite dilué avec de l'alcool isopropylique aqueux puis acidifié à l'acide sulfurique. On refroidit ensuite à 18-20 °C pour faire cristalliser le produit que l'on essore, lave avec une solution de soude puis à l'eau. On recristallise le produit de l'isopropanol. On obtient 605 g de fénofibrate (Rendement = 83,9 %) de pureté supérieure à 99,5 % (détermination par chromographie liquide haute pression, en abrégé HPLC).

Préparation II : (Exemple comparatif CP 1)

Dans un ballon à 3 cols de 1 litre équipé d'une agitation et d'un réfrigérant, on introduit 46,5 g

(0,2 mole) de (4-chlorophényl)-(4-hydroxyphényl)-méthanone, 35 g (0,25 mole) de carbonate de potassium et 400 ml de 4-méthyl-2-pentanone (autre nomenclature : méthylisobutylcétone). On porte à reflux pendant 2 heures pour former le sel de potassium de la (4-chlorophényl)-(4-hydroxyphényl)-méthanone, puis on ajoute 41,8 g (0,2 mole) de 1-méthyléthyl ester de l'acide 2-bromo-2-méthylpropanoïque. On porte à reflux pendant 12 heures. Après refroidissement, on filtre les sels minéraux insolubles et on concentre le filtrat sous pression réduite. Le résidu résultant est repris avec de l'éther éthylique, lavé avec une solution de soude à 4 % puis à l'eau. Après évaporation du solvant, le résidu est recristallisé de l'éther isopropylique. On obtient 20 g de fénofibrate (Rendement = 27,7 %).

Préparation III : (Exemple comparatif CP 2)

Dans un ballon à 3 cols de 500 ml équipé d'une agitation et d'un réfrigérant, on introduit 200 ml d'éthanol anhydre. On ajoute ensuite, par fractions, 4,6 g (0,2 atome-gramme) de sodium. Quand tout le sodium est dissous, on ajoute 46,5 g (0,2 mole) de (4-chlorophényl)-(4-hydroxyphényl)-méthanone et on porte à reflux pendant 30 minutes. On ajoute ensuite 41,8 g (0,2 mole) de 1-méthyléthyl ester de l'acide 2-bromo-2-méthylpropanoïque et on porte à reflux pendant 8 heures. Après concentration, le milieu réactionnel est traité de la même façon que dans la préparation II. Par recristallisation, on obtient 25 g de fénofibrate (Rendement = 34,7 %).

Préparation IV : (Exemple comparatif CP 3)

Dans un réacteur de 4 litres équipé d'une agitation et d'un réfrigérant, on introduit 1 litre d'alcool isopropylique, 232,5 g (1 mole) de (4-chlorophényl)-(4-hydroxyphényl)-méthanone, 138 g (1 mole) de carbonate de potassium et 355 g (1,7 mole) de 1-méthyléthyl ester de l'acide 2-bromo-2-méthylpropanoïque. On chauffe doucement le milieu réactionnel sous agitation vigoureuse, puis on maintient à reflux pendant 8 heures. On distille ensuite environ 400 ml d'alcool isopropylique, puis on refroidit le milieu sous agitation. Le précipité formé est essoré puis lavé sous agitation en phase hétérogène par de l'eau. On essore puis lave à nouveau avec une solution de soude à 2 %, puis par de l'eau jusqu'à neutralité. Le produit essoré est purifié par recristallisation de l'alcool isopropylique. On obtient 140 g de fénofibrate (Rendement = 38,8 %).

Préparation V : (Exemple comparatif CP 4)

Dans un ballon à 3 cols de 1 litre, on introduit 300 ml de diméthylformamide, 100 g (0,43 mole) de (4-chlorophényl)-(4-hydroxyphényl)-méthanone et 68,2 g de carbonate de potassium (0,49 mole). On porte à reflux du solvant pendant 0,5 h, sous agitation vigoureuse, puis on ajoute 120 g (0,57 mole) de 1-méthyléthyl ester de l'acide 2-bromo-2-méthylpropanoïque. On maintient à reflux pendant 4 heures. Après refroidissement, le milieu réactionnel est hydrolysé avec l'eau, puis extrait au chloroforme. La phase organique est ensuite lavée avec une solution de soude à 3 % en poids, puis à l'eau jusqu'à neutralité. Le résidu obtenu après évaporation du solvant est recristallisé de l'alcool isopropylique. On obtient 30 g de fénofibrate (Rendement = 19,3 %).

Préparation VI : (Exemple 1)

Obtention du 1-méthyléthyl ester de l'acide 2-(4-(4-chlorobenzoyl)-phénoxy)-2-méthylpropanoïque (fénofibrate)

Dans un ballon à 3 cols, équipé d'une agitation et d'un réfrigérant, on introduit sous atmosphère d'azote 100 g (0,43 mole) de (4-chlorophényl)-(4-hydroxyphényl)-méthanone, 165 g (0,79 mole) de 1-méthyléthyl ester de l'acide 2-bromo-2-méthylpropanoïque. On porte le milieu réactionnel à 110 °C puis, tout en distillant à 100 °C, on ajoute lentement en 20 minutes une solution de 50 g (0,36 mole) de carbonate de potassium dans 50 ml d'eau déminéralisée. Le distillat décante en 2 phases. La phase lourde est recyclée dans le milieu réactionnel. Après 1,5 h de chauffage à 110-112 °C, on porte le milieu réactionnel à 140 °C et on maintient pendant 4 heures une température de 140-145 °C. On refroidit ensuite le milieu réactionnel vers 90 °C et on ajoute 210 ml d'alcool isopropylique à 80 %. On laisse ensuite refroidir le mélange sous agitation pendant 12 h puis on filtre à 0 °C la suspension obtenue. Le précipité est lavé par 4 fois 200 ml d'eau déminéralisée, puis recristallisé du 2-propanol. On obtient ainsi 119,5 g (Rendement = 77 %) de fénofibrate.

Préparation VII : (Example 2)

Obtention de l'acide 2-(4-(2-((4-chlorobenzoyl)amino)éthyl) phénoxy)-2-méthylpropanoïque (bézafibrate)

1) Dans un ballon de 500 ml, équipé d'une agitation et d'un réfrigérant, on introduit sous atmosphère

d'azote 27,5 g (0,1 mole) de 4-(N-(4-chlorobenzoyl)-2-aminoéthyl)phénol, 38 g (0,18 mole) de 1-méthyléthyl ester de l'acide 2-bromo-2-méthylpropanoïque. On porte le milieu réactionnel à 135 °C puis on ajoute lentement 20 g (0,145 mole) de carbonate de potassium. On porte la température à 140-145 °C sous agitation pendant 4 h. On additionne alors 5 g (0,024 mole) de 1-méthyléthyl ester de l'acide 2-bromo-2-méthylpropanoïque et 5 g (0,036 mole) de carbonate de potassium. On maintient le milieu réactionnel à 145 °C pendant 1 h, puis on refroidit à 100 °C. On ajoute, sous agitation vigoureuse, 100 ml de 2-propanol, puis un mélange de 80 ml de 2-propanol, 6 ml d'acide sulfurique et 30 ml d'eau. On laisse refroidir et on essore le précipité formé. Par empâtages successifs avec une solution de soude à 1 %, puis lavage à l'eau jusqu'à neutralité, on obtient 43 g de produit que l'on recristallise du 2-propanol à 90 %. On obtient 36,4 g (Rendement = 90 %) de 1-méthyléthyl ester de l'acide 2-(4-(2-((4-chlorobenzoyl)amino)éthyl)phénoxy)-2-méthylpropanoïque fondant à 84 °C.

2) 36 g de l'ester obtenu précédemment sont hydrolysés dans 130 ml de méthanol, par 4,25 g de soude, à 50 °C pendant 1 h. Après concentration, le résidu est repris à l'eau. La phase aqueuse est lavée à l'éther, puis acidifiée à froid. L'acide attendu précipite. Après filtration, lavage à l'eau et séchage, on obtient 26 g (Rendement = 80 %) de bézafibrate fondant à 183 °C.

Préparation VIII : (Exemple 3)

Obtention de l'acide 2-(4-(2,2-dichlorocyclopropyl)phénoxy)-2-méthylpropanoïque (ciprofibrate)

1) On introduit dans un réacteur de 6 l, sous atmosphère d'azote, 1 500 g (11 moles) de méthyl-(4-hydroxyphényl)-méthanone et 3 800 g (18,2 moles) de 1-méthyléthyl ester de l'acide 2-bromo-2-méthylpropanoïque. On chauffe à 120 °C et on ajoute lentement 1 300 g (9,4 moles) de carbonate de potassium. Un mélange d'eau et de produits organiques distille. On élève la température à 140 °C. Après 1 heure, on additionne 350 g (1,7 mole) de 1-méthyléthyl ester de l'acide 2-bromo-2-méthylpropanoïque puis 222 g (1,6 mole) de carbonate de potassium. On maintient la température à 140 °C pendant 1 heure, puis on refroidit à 80 °C. On ajoute alors 4 litres de 2-propanol et on laisse refroidir sous agitation. On essore les sels minéraux insolubles et on concentre le filtrat sous pression réduite. Le résidu est repris à l'acétate d'éthyle, lavé avec une solution de soude à 10 % puis à l'eau. La phase organique est séchée, concentrée, et l'huile obtenue est distillée à 136-138 °C sous 0,5 mm de mercure. On obtient 2 350 g (Rendement = 81 %) de 1-méthyléthyl ester de l'acide 2-(4-acétylphénoxy)-2-méthylpropanoïque.

2) Dans un réacteur de 10 litres, on introduit, sous atmosphère d'azote, 2 350 g (8,9 moles) de l'ester obtenu précédemment et 3 litres de méthanol. On refroidit le milieu réactionnel à 0 °C et on ajoute lentement, sous agitation vigoureuse, 576,5 g (10,68 moles) de borohydrure de potassium. On maintient l'agitation pendant 12 h à température ambiante puis on concentre sous pression réduite. Le résidu est traité à l'eau glacée et repris à l'acétate d'éthyle. Après lavage à l'eau, la phase organique est séchée et concentrée. On obtient 2 355 g (Rendement = 99,5 %) de 1-méthyléthyl ester de l'acide 2-(4-(1-hydroxyéthyl) phénoxy)-2-méthylpropanoïque sous forme d'huile incolore.

3) On introduit dans un ballon de 1 litre, sous atmosphère d'azote, 240 ml de chloroforme, 120 g (0,453 mole) de l'ester obtenu précédemment et 3 ml de diméthylformamide. On refroidit le mélange à 0 °C puis, sous agitation, on introduit 18 ml de tribromure de phosphore en solution dans 50 ml de chloroforme. On maintient la température à 0 °C pendant 1 h. On agite ensuite le milieu réactionnel à 30 °C pendant 1 h, puis on ajoute 84 g de triéthylamine. On porte 8 h à reflux, puis on refroidit et on hydrolyse sur de la glace. On extrait au chloroforme et on filtre le mélange. Après lavage de la phase organique à l'eau puis séchage, on concentre sous pression réduite. On obtient 105 g (Rendement = 93 %) de 1-méthyléthyl ester de l'acide 2-(4-éthénylphénoxy)-2-méthylpropanoïque.

4) On introduit dans un ballon de 100 ml 5 g de l'ester obtenu précédemment, 12 ml de chloroforme puis 0,5 g de chlorure de benzyltriéthylammonium. On ajoute ensuite, goutte à goutte, 12 g de lessive de soude, puis on chauffe à 40 °C pendant 5 h. Le milieu réactionnel est ensuite refroidi, hydrolysé puis extrait au chloroforme. Après lavage à l'eau, la phase organique est séchée et concentrée sous pression réduite. On obtient 5 g (Rendement = 75 %) de 1-méthyléthyl ester de l'acide 2-(4-(2,2-dichlorocyclopropyl)phénoxy)-2-méthylpropanoïque sous forme d'huile.

5) On introduit dans un ballon de 100 ml 5 g de l'ester obtenu précédemment, 20 ml de méthanol et 0,84 g de soude. Sous agitation on chauffe à 50-60 °C pendant 2 h, puis on concentre sous pression réduite. Le solide obtenu est repris à l'eau et la solution aqueuse est lavée à l'éther puis acidifiée jusqu'à pH = 1 à l'aide d'acide chlorhydrique. On extrait à l'acétate d'éthyle. La phase organique est lavée à l'eau puis séchée et concentrée. L'huile obtenue cristallise par addition de cyclohexane. Le solide obtenu est recristallisé du toluène. On obtient 3,6 g (Rendement = 82 %) de ciprofibrate fondant à 115 °C.

Les préparations I-VIII données ci-dessus à titre d'illustration de l'invention et les exemples comparatifs mettent en évidence que le procédé selon l'invention permet d'obtenir

(i) des rendements très importants (83,9 %) comparativement à la technique antérieure faisant intervenir un solvant (19 % à 39 %) ;

(ii) des produits de très haute pureté requise dans la préparation d'un médicament ;

(iii) un gain d'énergie par réduction des durées de réaction (essentiellement réduction des temps de

chauffage) ;

(iv) une utilisation de solvant limitée aux cristallisations ;

(v) une unité opératoire plus importante pour un même volume de réacteur.

Le procédé selon l'invention est directement applicable à l'échelle industrielle.

Tableau I

| Exemples | Méthode (a) (Préparation) | Solvant | Produit obtenu | Rendement (%) |
|---|---|---|---|---|
| Ex 1 | A (I) | - | fénofibrate | 83,9 |
| CP 1 | B(II) | $CH_3COCH_2CH(CH_3)_2$ | fénofibrate | 27,7 |
| CP 2 | B(III) | $CH_3CH_2OH$ | fénofibrate | 34,7 |
| CP 3 | B (IV) | $CH_3CHOHCH_3$ | fénofibrate | 38,8 |
| CP 4 | B (V) | $HCON(CH_3)_2$ | fénofibrate | 19,3 |
| CP 5 | C | $CH_3CH_2OH$ | (b) | 76 |
| CP 6 | D ("acétone/chloroforme") | | fénofibrate | ≃ 70(c) |

Notes

(a) méthode :

A : selon l'invention par réaction de VI avec $BrC(CH_3)_2COOCH(CH_3)_2$ sans solvant ;

B : selon l'enseignement de GB-A-1 539 897 par réaction de VI avec $BrC(CH_3)_2COOCH(CH_3)_2$ en présence d'un solvant ;

C : selon l'enseignement de GB-A-1 539 897 par réaction de VI avec $BrCH(CH_3)COOCH_2CH_3$ en présence d'un solvant ;

D : selon l'enseignement de GB-A-1 539 897 par (i) réaction de VI avec le mélange acétone/chloroforme, puis (ii) estérification de l'acide correspondant.

(b) 2-[4-(4-chlorobenzoyl)-phénoxy]-propionate d'éthyle

(c) le rendement global de la méthode D est de 70 % environ ; plus précisément, on obtient l'acide fénofibrique avec un rendement de 85 % (cet acide contient 3 à 4 % en poids de phénol VI qui n'a pas réagi), puis l'estérification est effectuée avec un rendement de 85 %.

## Revendications

1. Procédé de préparation d'un composé choisi parmi l'ensemble des fibrates répondant à la formule générale

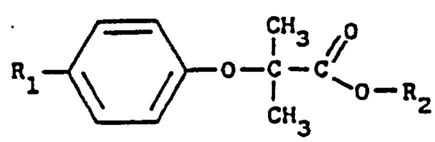

(I)

dans laquelle $R_1$ représente notamment un atome d'halogène (en particulier F, Cl, Br, l'atome d'halogène préféré étant Cl), un groupe acétyle, (4-chlorophényl)-hydroxyméthyle, 4-chlorobenzoyle ou 2-(4-chloro-benzamido)-éthyle ; et $R_2$ représente un groupe alkyle en $C_1$-$C_4$ dans lequel la chaîne hydrocarbonée est linéaire ou ramifiée, caractérisé en ce que l'on fait réagir, en l'absence de solvant, un excès, par rapport aux conditions stœchiométriques, de 2-bromo-2-méthylpropanoate d'alkyle de formule

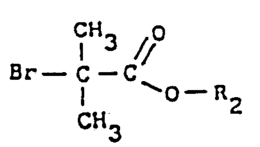

(V)

où $R_2$ est défini comme indiqué ci-dessus, avec un phénol substitué de formule

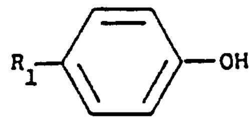

(VI)

où $R_1$ est défini comme indiqué ci-dessus, en présence d'un excès de $K_2CO_3$ par rapport aux conditions stœchiométriques, à une température supérieure ou égale à 120 °C, pendant au moins 2 h.

2. Procédé selon la revendication 1, caractérisé en outre par le fait que le fibrate ainsi obtenu est isolé du milieu réactionnel par précipitation, extraction ou distillation.

3. Procédé selon la revendication 1, caractérisé en outre par le fait que le milieu réactionnel contenant le fibrate ainsi obtenu est traité par un acide fort pour neutraliser l'excès de $K_2CO_3$, le fibrate étant ensuite isolé du milieu réactionnel par précipitation extraction ou distillation.

4. Procédé selon la revendication 1, caractérisé en ce que l'on fait réagir à une température de 120 à 160 °C, pendant 3 à 6 heures, 1 mole de VI avec environ 1,7 à environ 2,3 moles de V, en présence d'environ 0,8 à environ 1,8 moles de $K_2CO_3$.

5. Procédé selon la revendication 1, caractérisé en ce que l'on fait réagir à une température de 140 à 145 °C, 1 mole de VI avec environ 2 moles de V, en présence d'environ 1 mole de $K_2CO_3$.

6. Procédé selon la revendication 3, caractérisé en ce que la neutralisation de l'excès de $K_2CO_3$ est effectuée au moyen d'acide sulfurique à une température inférieure ou égale à 120 °C et de préférence voisine de 100 °C.

7. Procédé selon la revendication 1, caractérisé en ce que

1) l'on fait réagir, en l'absence de solvant pendant au moins 2 heures (de préférence pendant 3 à 6 heures), environ une mole de VI avec environ 1,7 à environ 2,3 moles de V (de préférence environ 2 moles de V), en présence de environ 0,8 à environ 1,8 moles de $K_2CO_3$ (de préférence environ 1 mole de $K_2CO_3$), à une température de 120 °C à 160 °C (de préférence à une température de 140 °C à 145 °C),

2) on neutralise l'excès $K_2CO_3$ au moyen d'un acide fort à une température inférieure à 120 °C, et

3) on isole le fibrate du milieu réactionnel par précipitation à une température inférieure à 60 °C, par extraction ou distillation.

8. Procédé selon la revendication 1, pour la préparation du fénofibrate, caractérisé en ce que

(a) l'on fait réagir, en l'absence de solvant pendant environ 5 heures, environ 1 mole de VI où $R_1$ est le groupe 4-chlorobenzoyle avec environ 2 moles de V où $R_2$ est le groupe isopropyle, en présence de environ 1 mole de $K_2CO_3$, à une température de environ 140 °C à environ 145 °C,

(b) après addition d'isopropanol aqueux au milieu réactionnel ainsi obtenu, on neutralise l'excès de $K_2CO_3$ au moyen d'acide sulfurique à une température voisine de 100 °C,

(c) on refroidit le milieu réactionnel résultant à une température comprise entre 15 et 25 °C, et recueille par filtration le précipité de fénofibrate,

(d) on lave le précipité ainsi filtré à la soude puis à l'eau, puis

(e) on recristallise le fénofibrate de l'isopropanol aqueux.

9. Procédé de préparation d'un fibrate de formule I selon la revendication 1, où $R_2 = H$, caractérisé en ce qu'on prépare l'ester correspondant selon le procédé de la revendication 1 par réaction du phénol substitué VI avec un 2-bromo-2-méthylpropanoate d'alkyle de formule V où $R_2$ est un groupe alkyle en $C_1$-$C_4$, en l'absence de solvant, puis saponifie l'ester résultant.

**Claims**

1. A method for the preparation of a compound selected from the group comprising the fibrates corresponding to the general formula

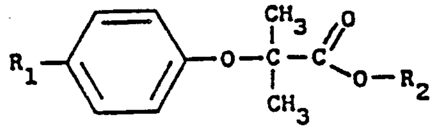

(I)

in which $R_1$ represents especially a halogen atom (in particular F, Cl or Br, the preferred halogen atom being Cl) or an acetyl, (4-chlorophenyl) hydroxymethyl, 4-chlorobenzoyl or 2-(4-chlorobenzamido) ethyl group and $R_2$ represents a $C_1$-$C_4$ alkyl group in which the hydrocarbon chain is linear or branched, which comprises reacting an excess, relative to the stoichiometric conditions, of an alkyl 2-bromo-2-methylpropanoate of the formula :

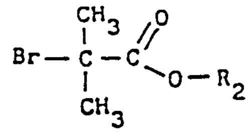

(V)

in which $R_2$ is defined as indicated above, with a substituted phenol of the formula

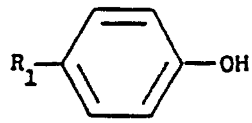

(VI)

in which $R_1$ is defined as indicated above, in the absence of a solvent and in the presence of an excess of $K_2CO_3$, relative to the stoichiometric conditions, at a temperature greater than or equal to 120 °C, for at least 2 h.

2. The method according to claim 1, wherein the resulting fibrate is isolated from the reaction medium by precipitation, extraction or distillation.

3. The method according to claim 1, wherein the reaction medium containing the resulting fibrate is treated with a strong acid to neutralize the excess $K_2CO_3$, and the fibrate is then isolated from the reaction medium by precipitation, extraction or distillation.

4. The method according to claim 1, wherein 1 mol of VI is reacted with about 1.7 to about 2.3 mol of V in the presence of about 0.8 to about 1.8 mol of $K_2CO_3$, at a temperature of 120 to 160 °C, for 3 to 6 hours.

5. The method according to claim 1, wherein 1 mol of VI is reacted with about 2 mol of V in the presence of about 1 mol of $K_2CO_3$, at a temperature of 140 to 145 °C.

6. The method according to claim 3, wherein the neutralization of the excess $K_2CO_3$ is carried out with sulfuric acid at a temperature not exceeding 120 °C and preferably of the order of 100 °C.

7. The method according to claim 1, wherein :

1) about one mol of VI is reacted with about 1.7 to about 2.3 mol of V (preferably about 2 mol of V) in the absence of a solvent and in the presence of about 0.8 to about 1.8 mol of $K_2CO_3$ (preferably about 1 mol of $K_2CO_3$), at a temperature of 120 °C to 160 °C (preferably at a temperature of 140 °C to 145 °C), for at least 2 hours (preferably for 3 to 6 hours),

2) the excess $K_2CO_3$ is neutralized with a strong acid at a temperature below 120 °C, and strong acid at a temperature below 120 °C, and

3) the fibrate is isolated from the reaction medium by precipitation at a temperature below 60 °C, or by extraction or distillation.

8. The method according to claim 1 for the preparation of fenofibrate, wherein :

(a) about 1 mol of VI in which $R_1$ is the 4-chlorobenzoyl group is reacted with about 2 mol of V in which $R_2$ is the isopropyl group, in the absence of a solvent and in the presence of about 1 mol of $K_2CO_3$, at a temperature of about 140 °C to about 145 °C, for about 5 hours,

(b) after the addition of aqueous isopropanol to the resulting reaction medium, the excess $K_2CO_3$ is neutralized with sulfuric acid at a temperature of the order of 100 °C,

(c) the resulting reaction medium is cooled to a temperature of between 15 and 25 °C and the precipitate of fenofibrate is collected by filtration,

(d) the precipitate filtered off in this way is washed with sodium hydroxide followed by water, and then

(e) the fenofibrate is recrystallized from aqueous isopropanol.

9. The method for the preparation of a fibrate of the formula I according to claim 1, in which $R_2 = H$, wherein the corresponding ester is prepared, according to the method of claim 1, by reacting the substituted phenol VI with an alkyl 2-bromo-2-methyl-propanoate of the formula V in which $R_2$ is a $C_1$-$C_4$ alkyl group, in the absence of a solvent, and the resulting ester is then saponified.

## Patentansprüche

1. Verfahren zur Herstellung einer Verbindung aus der Gruppe der Fibrate der allgemeinen Formel

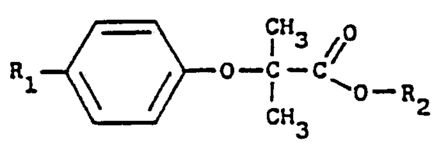

(I)

in welcher $R_1$ in erster Linie ein Halogenatom (insbesondere F, Cl, Br, als bevorzugtes Halogenatom : Cl), eine Acetyl-Gruppe, (4-Chlorophenyl)-hydroxymethyl, 4-Chlorobenzoyl oder 2-(4-Chlorobenzamido)-ethyl ist und $R_2$ Alkylgruppe mit $C_1$ bis $C_4$ darstellt, in welcher die Kohlenwasserstoffkette geradkettig oder verzweigt ist, dadurch gekennzeichnet, daß bei Abwesenheit von Lösungsmittel ein stöchiometrischer Überschuß von Alkyl-2-bromo-2-methylpropanoat der Formel

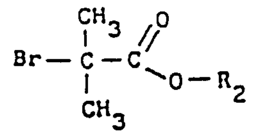

(V)

worin $R_2$ die oben angegebene Bedeutung hat, mit einem substituierten Phenol der Formel

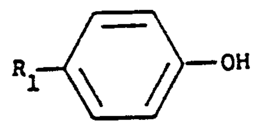

(VI)

worin $R_1$ die oben angegebene Bedeutung hat, in Gegenwart eines stöchiometrischen Überschusses von $K_2CO_3$ bei einer Temperatur von mindestens 120 °C mindestens 2 Std. lang zur Reaktion gebracht wird.

2. Verfahren nach Anspruch 1, weiter dadurch gekennzeichnet, daß das auf diese Weise gewonnene Fibrat aus dem Reaktionsmedium durch Abscheidung, Extraktion oder Destillation isoliert wird.

3. Verfahren nach Anspruch 1, weiter dadurch gekennzeichnet, daß das Reaktionsmedium, das das auf diese Weise gewonnene Fibrat enthält, zum Neutralisieren des Überschusses von $K_2CO_3$ mit einer starken Säure behandelt wird, und daß das Fibrat danach durch Abscheidung, Extraktion oder Destillation aus dem Reaktionsmedium isoliert wird.

4. Verfahren nach Anspruch 1, dadurch gekennzeichnet, daß bei einer Temperatur von 120 bis 160 °C 3 bis 6 Std. lang 1 Mol von (VI) mit etwa 1,7 etwa 2,3 Mol von (V) in Gegenwart von etwa 0,8 bis etwa 1,8 Mol $K_2CO_3$ umgesetzt wird.

5. Verfahren nach Anspruch 1, dadurch gekennzeichnet, daß bei einer Temperatur von 140 bis 145 °C 1 Mol von (VI) mit etwa 2 Mol von (V) in Gegenwart von etwa 1 Mol $K_2CO_3$ umgesetzt wird.

6. Verfahren nach Anspruch 3, dadurch gekennzeichnet, daß die Neutralisierung des $K_2CO_3$-Überschusses mittels Schwefelsäure bei einer Temperatur von höchstens 120 °C, vorzugsweise bei nahe 100 °C, vorgenommen wird.

7. Verfahren nach Anspruch 1, dadurch gekennzeichnet, daß

1) bei Abwesenheit von Lösungsmittel mindestens 2 Std. lang (vorzugsweise 3 bis 6 Std. lang) etwa 1 Mol von (VI) mit etwa 1,7 bis etwa 2,3 Mol von (V) (vorzugsweise etwa 2 Mol von (V)) in Gegenwart von etwa 0,8 bis etwa 1,8 Mol $K_2CO_3$ (vorzugsweise etwa 1 Mol $K_2CO_3$) bei einer Temperatur von 120 bis 160 °C (vorzugsweise bei einer Temperatur von 140 bis 145 °C) umgesetzt wird,

2) der $K_2CO_3$-Überschuß mittels einer starken Säure bei einer Temperatur von weniger als 120 °C neutralisiert wird, und

3) das Fibrat aus dem Reaktionsmedium durch Abscheidung bei einer Temperatur von weniger als 60 °C, durch Extraktion oder durch Destillation isoliert wird.

8. Verfahren nach Anspruch 1 zur Herstellung des Fenofibrats, dadurch gekennzeichnet, daß

(a) bei Abwesenheit von Lösungsmittel etwa 5 Std. lang etwa 1 Mol von (VI), worin $R_1$ die 4-Chlorobenzoyl-Gruppe ist, mit etwa 2 Mol von (V), worin $R_2$ die Isopropyl-Gruppe ist, in Gegenwart von etwa 1 Mol $K_2CO_3$ bei einer Temperatur von etwa 140 °C bis etwa 145 °C umgesetzt wird,

(b) nach Zugabe von wasserhaltigem Isopropanol zu dem so gewonnenen Reaktionsmedium der $K_2CO_3$-Überschuß mit Schwefelsäure bei einer Temperatur nahe 100 °C neutralisiert wird,

(c) das entstandene Reaktionsmedium bis auf eine Temperatur zwischen 15 und 25 °C gekühlt und der Fenofibrat-Niederschlag durch Filtration aufgefangen wird,

(d) der so filtrierte Niederschlag mit Natronlauge und danach mit Wasser gewaschen wird und dann

(e) das Fenofibrat aus dem wasserhaltigen Isopropanol umkristallisiert wird.

9. Verfahren zur Herstellung eines Fibrats der Formel I nach Anspruch 1, worin $R_2 = H$, dadurch gekennzeichnet, daß der entsprechende Ester nach dem Verfahren von Anspruch 1 hergestellt wird durch Umsetzen des substituierten Phenols (VI) mit einem Alkyl-2-bromo-2-methylpropanoat der Formel (V), worin $R_2$ eine Alkylgruppe mit $C_1$ bis $C_4$ ist, in Abwesenheit von Lösungsmittel und anschließendes Verseifen des entstehenden Esters.